# EUROPEAN PATENT SPECIFICATION

(11) **EP 3 005 878 B1**
(45) Date of publication and mention of the grant of the patent: **27.02.2019**
(21) Application number: 15186230.7
(22) Date of filing: 22.09.2015
(51) Int. Cl.: A23D 9/013, A23J 7/00, A61K 8/55, A61K 8/36, A61Q 19/00, A23K 20/158, A23L 29/10, A23L 5/41

(54) **A METHOD FOR SUPPRESSING HEAT DISCOLORATION OF LECITHIN**
VERFAHREN ZUR UNTERDRÜCKUNG VON WÄRMEENTFÄRBUNG VON LECITHIN
PROCÉDÉ DE SUPPRESSION DE LA DÉCOLORATION THERMIQUE DE LA LÉCITHINE

(30) Priority: 07.10.2014 JP 2014206771
(43) Date of publication of application: 13.04.2016
(73) Proprietor: TSUJI OIL MILLS CO., LTD., Mie Matsusaka-shi 515-2314 (JP)
(72) Inventor: Fujimoto, Yuki, Mie, 515-2314 (JP); Hayashi, Akihito, Mie, 515-2314 (JP); Hamaguchi, Nobutoshi, Mie, 515-2314 (JP)
(74) Representative: Witte, Weller & Partner Patentanwälte mbB

(56) References cited:
- WO-A1-2006/015841
- GB-A- 1 307 718
- JP-A- S54 120 607
- US-A- 2 839 546
- US-A- 4 524 085
- US-A- 5 374 434
- US-A1- 2002 192 295
- US-A1- 2004 120 908
- US-A1- 2010 209 588

## Description

### Technical Field

The present invention relates to a method for suppressing heat discoloration of lecithin.

### Background art

Lecithin is a generic name for a mixture mainly comprising various phospholipids, and the major components thereof are phosphatidylcholine (PC), phosphatidylethanolamine (PE), phosphatidylinositol (PI), phosphatidylserine (PS), phosphatidylglycerol (PG), phosphatidic acid (PA), and acyl glycero phospholipids including lysophospholipids derived from these phospholipids by hydrolysis of a fatty acid at the sn-1 or the sn-2 position. Lecithin is broadly present in living organisms such as animals, plants, and microorganisms, and is particularly contained much in brains and livers of animals, egg yolks, soybeans, yeasts, and the like. Lecithin is broadly used as a natural emulsifier for foods, industrial products, cosmetics, medicines, and the like. Since lecithin is excellent in preventive effect on oil splattering caused by other ingredients and mold release effect, known examples of edible oils and fats for which lecithin is used include a stir-frying oil, a mold release oil, a fried rice oil, a frying oil, and the like, which are edible oils and fats prepared by addition and dissolution of lecithin. However, when a lecithin-containing oil or fat is heated (at a temperature of 100°C or more), the oil or fat gradually turns brownish yellow, brown, and almost black in the end. Accordingly, when a lecithin-containing oil or fat is used for a stir-frying oil for example, discoloration by heating occurs, leading to problems such as bad appearance of stir-fried dishes. Therefore, the development of the technique by which the heat discoloration of a lecithin-containing oil or fat in use is suppressed is desired.

As a method for suppressing the heat discoloration of lecithin, a method in which an additive for suppressing the discoloration is used has been developed. For example, Patent Literature 1 discloses a method in which a carbonate is added to a phospholipid-containing oil or fat and Patent Literature 2 discloses a method in which sodium acetate is added to a phospholipid-containing oil or fat. However, in recent years, the quality level of confectionery, bread, and delicatessen food has become high, and the number of foods containing large amounts of eggs, sugar, or the like and being likely to be discolored has been increasing. Therefore, studies have been conducted to find a method for producing a lecithin-containing oil or fat having an enhanced discoloration resistance compared to those of traditional lecithin-containing oils and fats. For example, Patent Literature 3 discloses a method for suppressing the heat discoloration of a lecithin-containing oil or fat, in which a polyglycerin-condensed ricinoleic acid ester is added thereto.

WO 2006/015841 A, US 2002/192295 A1, US 2004/120908 A1, US 5,374,434 A and US 2010/209588 A1 disclose all examples of lecithin preparations.

US 2,839,546 A discloses the treatment of bleached phosphatides with metal salts. GB 1307718 A discloses sterile lecithin and its preparation. US 4,524,085 A discloses lecithin containing cooking fats with reduced thermal discoloration. JP S54120607 A discloses the prevention of heat browning of phosphor lipids by adding amino acids to the oil or fat containing phosphor lipid.

### Citation List

### Patent Literature

PTL 1: JP 54-110210 A
PTL 2: JP 54-120609 A
PTL 3: JP 2007-68462 A

### Summary of Invention

### Technical Problem

An objective of the present invention is to provide a novel method for suppressing heat discoloration of lecithin.

### Solution to Problem

In order to solve the problem, the present invention encompasses the inventions below.
[1] A method for suppressing heat discoloration of lecithin, comprising adding a fatty acid metal salt to lecithin.
[2] The method for suppressing heat discoloration according to the above [1], wherein the metal constituting the fatty acid metal salt is at least one selected from the group consisting of lithium, beryllium, sodium, magnesium, aluminum, potassium, calcium, iron, cobalt, nickel, copper, zinc, silver, barium
   thallium, and lead.
[3] The method for suppressing heat discoloration according to the above [1] or [2], wherein the fatty acid of the fatty acid metal salt is at least one selected from saturated fatty acids and unsaturated fatty acids having 3 to 36 carbon atoms.
[4] Use of a fatty acid metal salt for producing a heat discoloration suppressant for lecithin.

### Advantageous Effects of Invention

The present invention can provide a novel method for suppressing heat discoloration of lecithin. Since the present invention, in which a fatty acid metal salt is added to lecithin, is simple in terms of constitution, the method for suppressing heat discoloration can be performed easily and the lecithin or lecithin preparation of the present disclosure having resistance to heat discoloration can be produced at low cost. Moreover, according to the present invention, heat discoloration can be suppressed without impairing the original functions of lecithin. The use of an edible oil or fat containing the lecithin or lecithin preparation of the present disclosure having resistance to heat discoloration, for example, for a stir-frying oil, a mold release oil, a frying oil, or the like can provide a high-quality processed food having suppressed heat discoloration of lecithin.

### Brief Description of Drawings

FIG. 1 shows the results of heat discoloration tests in which various fatty acid metal salts were added separately to a soybean lecithin paste.
FIG. 2 is pictures showing the appearances of samples after the heat discoloration tests in which various fatty acid metal salts were added separately to a soybean lecithin paste.
FIG. 3 shows the results of heat discoloration tests in which a fatty acid metal salt was added to a high-purity soybean lecithin powder.
FIG. 4 shows the results of heat discoloration tests in which a fatty acid metal salt was added to a PC-concentrated soybean lecithin paste.
FIG. 5 shows the results of heat discoloration tests in which a fatty acid metal salt was added to a PC-concentrated soybean lecithin lump.

### Description of Embodiments

The present invention provides a method for suppressing heat discoloration of lecithin (hereinafter referred to as "the method of the present invention"). The method of the present invention is characterized in that a fatty acid metal salt is added to lecithin. The present disclosure also provides a lecithin or lecithin preparation having resistance to heat discoloration. The lecithin or lecithin preparation of the present disclosure is characterized by containing a fatty acid metal salt.

Lecithin is a generic name for a mixture mainly comprising various phospholipids. Examples of the phospholipid which is a major component of lecithin include phosphatidylcholine (PC), phosphatidylethanolamine (PE), phosphatidylinositol (PI), phosphatidylserine (PS), phosphatidylglycerol (PG), phosphatidic acid (PA), and acyl glycero phospholipids including lysophospholipids derived from these phospholipids by hydrolysis of a fatty acid at the sn-1 or the sn-2 position. A single component selected from the above phospholipids or a mixture of two or more kinds thereof may be referred to as lecithin. For industrial purposes, mixtures with a phospholipid purity of 60% by mass or more are used as lecithin. The phospholipid purity can be calculated by subtracting the weight of toluene-insoluble matter and acetone-soluble matter from the weight of a mixture, taking advantage of the property that a phospholipid is dissolved in toluene easily and not in acetone. Lecithin includes a fractionated lecithin, which is obtained by subjecting a lecithin to solvent fractionation; an enzyme-degraded lecithin or an enzyme-treated lecithin, which is obtained by subjecting a lecithin to enzyme treatment; a hydrogenated lecithin, which is obtained by subjecting a lecithin to hydrogenation; an acetylated lecithin, which is obtained by subjecting a lecithin to acetylation; a hydroxylated lecithin, which is obtained by subjecting a lecithin to hydroxylation; and a lecithin obtained by a combination of solvent fractionation, enzyme treatment, hydrogenation, acetylation, and/or hydroxylation. The form of the lecithin is not particularly limited, and may be any form such as a powder, a paste, or a lump.

Lecithin preparation is a generic name for a mixture of a lecithin as a main active ingredient and an auxiliary agent added for convenience in use. Examples of the auxiliary agent include food additives such as a manufacturing agent, an enzyme, a pH adjuster, a preservative, a sterilizer, an antioxidant, an antifungal agent, a shelf life improver, a colorant, a color improver, a decolorant, a brightener, a flavor, a spice extract, a sweetener, an acidulant, a seasoning, a bittering agent, an emulsifier, a thickener, a stabilizer, a gelatinizer, an adhesive paste, a leavening agent, a gum base, a yeast food, a softener, and an enrichment; food materials such as a lipid, a carbohydrate, a processed starch, a protein, and a peptide; and water. The auxiliary agents may be used alone or in combination of two or more kinds thereof. The form of the lecithin preparation is not particularly limited, and may be any form such as a powder, a paste, and a lump.

The source material of the lecithin is not particularly limited, and preferred examples thereof include, plants, animals, and aquatic animals and plants. Specific examples of the lecithin derived from a plant include lecithins obtained from a by-product (for example, a hydrate generated in the degumming process)of the purification of a vegetable oil of tung, linseed, almond, inca inchi, perilla, olive, orange seed, pumpkin seed, kapok, mustard, *Trichosanthes kirilowii* seed, *Catalpa ovata* seed, *Calendula officinalis* seed, wheat germ, rice bran, corn, sesame, cherry seed, safflower, pomegranate seed, *Perilla frutescens*, snakeguard seed, soybean, tea seed, evening primrose seed, camellia, rapeseed, *Momordica charantia* seed, *Campsis grandiflora* seed, balsam apple seed, palm, sunflower, peanut, grape seed, *Impatiens balsamina* seed, macadamia nut, cottonseed, and ground nut. Specific examples of the lecithin derived from an animal include egg-yolk lecithin. Specific examples of the lecithin derived from an aquatic animal include lecithins obtained from sardine, salmon, mackerel, saury, herring, tuna, squid, *Alaska Pollack*, bonito, seal, krill, sand eel, and salmon roe.

The fatty acid metal salt is not particularly limited, and any fatty acid metal salt can be appropriately used. The fatty acid constituting the fatty acid metal salt is not particularly limited, and for example, a saturated or unsaturated, linear or branched fatty acid can be used. Moreover, the fatty acid constituting the fatty acid metal salt may have a substituent. The substituent is not particularly limited, and examples thereof include a hydroxyl group, a peroxy group, a carboxyl group, a ketone group, a aldehyde group, a carbonyl group, a nitro group, an amino group, a sulfo group, an imino group, a cyano group, an azo group, an azide group, a thiol group, a nitro group, an ether linkage, an ester linkage, an amide linkage, an urethane linkage, an alkyl group, a cycloalkyl group, and an alkoxy group. The number of the carbon atoms of the fatty acid is preferably 3 to 36, more preferably 6 to 30, and further preferably 8 to 22. Specific examples thereof include propionic acid, butyric acid, valeric acid, caproic acid, enanthic acid, caprylic acid, pelargonic acid, capric acid, lauric acid, myristic acid, pentadecylic acid, palmitic acid, palmitoleic acid, margaric acid, stearic acid, tuberculostearic acid, isostearic acid, oleic acid, elaidic acid, vaccenic acid, linoleic acid, α-linolenic acid, γ-linolenic acid, eleostearic acid, stearidonic acid, ricinoleic acid, arachidic acid, arachidonic acid, dihomo-γ-linolenic acid, eicosapentaenoic acid, behenic acid, erucic acid, docosapentaenoic acid, docosahexaenoic acid, lignoceric acid, nervonic acid, cerotic acid, montanoic acid, melissic acid, malonic acid, succinic acid, malic acid, citric acid, glutaric acid, adipic acid, pimelic acid, suberic acid, azelaic acid, and sebacic acid.

The metal constituting the fatty acid metal is not particularly limited, and, for example, metal elements of groups 1 to 14 in the periodic table, excluding hydrogen and carbon, can be used. Among them, alkali metals, alkali earth metals, and transition metals are more preferred and alkali metals and alkali earth metals are further preferred. Specific examples thereof include lithium, beryllium, sodium, magnesium, aluminum, potassium, calcium, iron, cobalt, nickel, copper, zinc, silver, barium, thallium, and lead. More preferred are sodium, magnesium, aluminum, potassium, calcium, iron, nickel, silver, and barium. Further preferred are sodium, magnesium, aluminum, potassium, calcium, and barium.

The method of the present invention can be performed by making a fatty acid metal salt and lecithin exist in the lecithin-containing object to be heated, in a state where they can contact with each other. The timing of the addition of the fatty acid metal salt to lecithin is not particularly limited. The fatty acid metal salt may be added to lecithin before or after heating is started. In the case where the fatty acid metal salt is added before the start of the heating, the salt may be added just before the start of the heating, and it is also allowable to use a fatty acid metal salt-containing lecithin, which is obtained by adding a fatty acid metal salt to lecithin. In the case where the fatty acid metal salt is added after the start of the heating, the salt is added preferably before the temperature of lecithin reaches the temperature at which lecithin discolors. Specifically, the addition is preferably performed before the temperature of the object being heated reaches 150°C, more preferably before the temperature reaches 130°C, and further preferably before the temperature reaches 100°C.

The amount of the fatty acid metal salt is not particularly limited, and the fatty acid metal salt may be added in an amount selected as appropriate so that a heat discoloration suppression effect can be exhibited. Preferably, the amount is about 0.01% by mass or more relative to the mass of the phospholipids contained in the lecithin of the object to be heated. About 0.1% by mass or more is more preferred and about 1% by mass or more is further preferred. There is no upper limit to the amount, but the amount is preferably about 5000% by mass or less relative to the mass of the phospholipids contained in the lecithin of the object to be heated, more preferably about 500% by mass or less, and further preferably about 100% by mass or less.

The lecithin-containing object to be heated is not particularly limited. Anything can be the object to be heated as long as it contains lecithin and the heat discoloration thereof can be suppressed by the method of the present invention. The object to be heated may be lecithin alone and may contain a material other than lecithin. Examples of the object containing a material other than lecithin include the lecithin preparation described above, a lecithin-containing oil or fat, a lecithin-containing food additive, a lecithin-containing food or drink, a lecithin-containing cosmetic, a lecithin-containing medicine, a lecithin-containing feed, and a lecithin-containing industrial product. The heat discoloration suppression can be visually confirmed.

The lecithin or lecithin preparation of the present disclosure having resistance to heat discoloration (hereinafter referred to as "the lecithin of the present disclosure") may be any lecithin or lecithin preparation as long as it contains a fatty acid metal salt. The content of the fatty acid metal salt is not particularly limited as long as the content is sufficient to exhibit the heat discoloration suppression effect. The content may be selected as appropriate based on the degree of the effect checked beforehand. Preferably, the amount is about 0.01% by mass or more relative to the mass of the phospholipids contained in the lecithin of the object to be heated. About 0.1% by mass or more is more preferred and about 1% by mass or more is further preferred. There is no upper limit to the amount, but the amount is preferably about 5000% by mass or less relative to the mass of the phospholipids contained in the lecithin of the object to be heated, more preferably about 500% by mass or less, and further preferably about 100% by mass or less.

The lecithin of the present disclosure can be produced by adding a fatty acid metal salt to lecithin and mixing the mixture. Specifically, a fatty acid metal salt is added to, for example, a lecithin powder, a lecithin paste, or a lecithin lump and the fatty acid metal salt is dispersed in the lecithin to produce the lecithin of the present disclosure. Preferably, the fatty acid metal salt is homogeneously dispersed in the lecithin.

The lecithin of the present disclosure may also be produced by a method in which lecithin is dissolved in a solvent or dispersed in a dispersion medium to prepare a lecithin solution or a lecithin dispersion and a fatty acid metal salt is added to the solution or the dispersion and mixed. Alternatively, the lecithin may be produced by a method in which a fatty acid metal salt is dissolved in a solvent or dispersed in a dispersion medium and then lecithin is added to the solution or the dispersion. Moreover, the lecithin solution or lecithin dispersion containing a fatty acid metal salt may be concentrated and/or dried. Furthermore, the lecithin solution or the lecithin dispersion may be purified to obtain a fractionated lecithin, which is obtained by subjecting lecithin of the present disclosure to solvent fractionation; an enzyme-degraded lecithin or an enzyme-treated lecithin, which is obtained by subjecting the lecithin to enzyme treatment; a hydrogenated lecithin, which is obtained by subjecting the lecithin to hydrogenation; an acetylated lecithin, which is obtained by subjecting the lecithin to acetylation; a hydroxylated lecithin, which is obtained by subjecting the lecithin to hydroxylation; or a lecithin obtained by a combination of solvent fractionation, enzyme treatment, hydrogenation, acetylation, and/or hydroxylation. The resulting lecithin and an auxiliary agent can be mixed to prepare a lecithin preparation. The lecithin of the present disclosure produced in this manner can be stably stored under the same conditions as those for an ordinary lecithin and an ordinary lecithin preparation.

The solvent used for dissolving the lecithin may be any solvent which can dissolve lecithin. Examples thereof include organic solvents such as a carboxylic acid alkyl ester, an alkane, an aliphatic hydrocarbon, an alicyclic hydrocarbon, an aromatic hydrocarbon, a halogenated hydrocarbon, and an alcohol. These solvents may be used alone or in combination of two or more kinds thereof. The dispersion medium used for dispersing the lecithin may be any dispersion medium which can disperse lecithin, and examples thereof include oils and fats derived from plants, animals, aquatic animals and plants, and microorganisms. These dispersion media may be used alone or in combination of two or more kinds thereof. Among the solvents and dispersion media, oils and fats can be preferably used. When such an oil or fat is used, there is no need of, for example, removing the dispersion medium, and the lecithin-containing oil or fat can be provided as it is. Accordingly the lecithin-containing oil or fat having resistance to heat discoloration can be produced in fewer steps, and the cost can be reduced.

The present disclosure provides an oil or fat containing the lecithin of the present disclosure. The oil or fat is not limited to edible oils and fats, and includes oils and fats used for other uses (for example, cosmetics, medicines, feeds, and industrial products) . Edible oils and fats are preferred. The edible oil or fat is not particularly limited, and a publicly known edible oil or fat derived from plants, animals, aquatic animals, microorganisms, and the like may be appropriately used. Specific examples of the oil or fat derived from a plant include tung oil, linseed oil, almond oil, inca inchi oil, perilla oil, olive oil, orange seed oil, pumpkin seed oil, kapok oil, mustard oil, *Trichosanthes kirilowii* seed oil, *Catalpa ovata* seed oil, a conjugated linoleic acid-containing oil or fat, *Calendula officinalis* seed oil, wheat germ oil, rice bran oil, corn oil, sesame oil, cherry seed oil, safflower oil, pomegranate seed oil, *Perilla frutescens* oil, snakeguard seed oil, soybean oil, tea seed oil, evening primrose seed oil, camellia oil, rapeseed oil, *Momordica charantia* seed oil, *Campsis grandiflora* seed oil, balsam apple seed oil, palm oil, sunflower oil, peanut oil, grape seed oil, *Impatiens balsamina* seed oil, macadamia nut oil, cottonseed oil, and ground nut oil. Specific examples of the oil or fat derived from an animal include beef tallow, lard, and egg yolk oil. Specific examples of the oil or fat derived from an aquatic animal include fish body oils obtained from sardine, salmon, mackerel, saury, herring, tuna, and other fishes, liver oils of squid and *Alaska Pollack*, orbital oils of bonito, tuna, and the like, seal oil, and krill oil. Specific examples of the oil or fat derived from a microorganism include an oil derived from *Schizochytrium sp*., an oil derived from *Nitzschia sp.*, an oil derived from *Nannochloris sp.*, and an oil derived from *Mortierella sp.* As a matter of course, a mixed oil or fat comprising two or more kinds of the above oils and fats, a hydrofined oil, a fractionated oil, a transesterified oil, and the like may also be used.

The lecithin content in the edible oil or fat is not particularly limited, and may be, for example, preferably 0.01 to 30% by mass, more preferably 0.5 to 15% by mass, and still more preferably 0.5 to 5.0% by mass.

The edible oil or fat of the present disclosure may be appropriately used for a stir-frying oil, a mold release oil, a fried rice oil, a frying oil, an oil or fat for noodles, an oil or fat for bread making, an oil or fat for confectionery, a flavor oil, and the like. The use of the edible oil or fat of the present disclosure for cooking can provide a high-quality food having suppressed heat discoloration.

The present disclosure provides food additives containing the lecithin of the present disclosure. Lecithin is used for food additives as a dispersing agent or an emulsifier for an oil-soluble component or a water-soluble component. In the process of producing food additives, the amount of a conventional lecithin is limited because heating and sterilization performed in the process cause heat discoloration of the lecithin. However, the use of the lecithin of the present disclosure can solve the problem of heat discoloration, and a food additive good in flavor and a preparation thereof can be prepared. Examples of the food additive include a manufacturing agent, an enzyme, a pH adjuster, a preservative, a sterilizer, an antioxidant, an antifungal agent, a shelf life improver, a colorant, a color improver, a decolorant, a brightener, a flavor, a spice extract, a sweetener, an acidulant, a seasoning, a bittering agent, an emulsifier, a thickener, a stabilizer, a gelatinizer, an adhesive paste, a leavening agent, a gum base, a yeast food, a softener, an enrichment, and preparations thereof.

The present disclosure provides cosmetics containing the lecithin of the present disclosure. Lecithin is used for cosmetics as a dispersing agent or an emulsifier for an oil-soluble component. In the process of producing cosmetics, the amount of a conventional lecithin is limited because heating and sterilization performed in the process cause heat discoloration of the lecithin. However, the use of the lecithin of the present disclosure can solve the problem of heat discoloration, and a cosmetic of which color is lighter compared to that of a cosmetic containing a conventional lecithin can be produced. Cosmetics include a so-called medicated cosmetic (quasi drug). Examples of the cosmetic include a cleanser, a shampoo, a conditioner, a hair tonic, a hair lotion, an after-shave lotion, a body lotion, a cosmetic lotion, a cleansing cream, a massage cream, an emollient cream, an aerosol product, an air refresher, an aromatic, a deodorant, and a bath additive. The cosmetic of the present disclosure may contain, in addition to the lecithin of the present disclosure, components typically used for cosmetics, such as a surface-active agent, a humectant, an oil or fat derived from an animal and a plant, an oil or fat derived from a microorganism, silicones, a higher alcohol, a lower alcohol, an extract derived from an animal and a plant, an extract derived from a microorganism, an ultraviolet absorber, an antiphlogistic, a sequestering agent, vitamins, an antioxidant, a thickener, a preservative, a disinfectant, a pH adjuster, a colorant, and a range of flavors as appropriate in accordance with a purpose.

The present disclosure provides a medicine containing the lecithin of the present disclosure. Although lecithin is used as an emulsifier for medicines, its use has been limited in some cases where heating is performed in the production process. However, the use of the lecithin of the present disclosure can solve the problem of heat discoloration. The medicine contains active ingredients in addition to the lecithin of the present disclosure and may further contain pharmaceutically acceptable carriers and additives as appropriate to give a formulation. In particular, the medicine may be formulated into oral preparations such as a tablet, a coated tablet, a pill, a powder, a granule, a capsule, a liquid, a suspension, and an emulsion, and parenteral preparations such as an injection, an infusion solution, a suppository, an ointment, and a patch. The blending ratio of a carrier or an additive may be set appropriately based on the range typically adopted in the pharmaceutical field. The carrier or the additive which can be contained is not particularly limited, and examples thereof include various carriers such as water, physiological saline, other aqueous solvents, and an aqueous or oily base; and various additives such as an excipient, a binder, a pH adjuster, a disintegrant, an absorption promoter, a lubricant, a colorant, a flavoring agent, and a flavor.

The present disclosure provides a feed containing the lecithin of the present disclosure. Although lecithin is used as an emulsifier for feeds or used for imparting a physiological function such as the improvement of lipid metabolism to feeds, its use has been limited in some cases where heating is performed in the production process. However, the use of the lecithin of the present disclosure can solve the problem of heat discoloration. Examples of the feed include a feed for livestock such as a cow, a horse, and a pig; a feed for poultry such as a chicken; a feed for cultured fish and shellfish; and a feed for pet animals such as a dog and a cat. The feed of the present disclosure may be processed and manufactured by a general method for producing feeds, except for the addition of the lecithin of the present disclosure to the feeds.

The present disclosure provides an industrial product containing the lecithin of the present disclosure. Although lecithin is used as a surface-active agent, an antioxidant, a release agent, and the like, for industrial products, its use has been limited in some cases where heating is performed. However, the use of the lecithin of the present disclosure can solve the problem of heat discoloration. Examples of the industrial product include coating materials (such as a paint, a varnish, a lacquer, an enamel, an ink, a photosensitizing agent, and a car wax), petroleum products (such as a lubricant, a grease, a cutting oil, a fuel oil, and a brake oil), agricultural chemicals (such as an antifungal agent and a control agent), resin products (such as a rubber and a plastic), magnetic products (such as a magnetic card and a magnetic tape), a leather product, and a fabric.

The present disclosure provides a food or drink containing the above edible oil or fat and/or the above food additive of the present disclosure. The food or drink includes a health food, a functional food, a food for specified health use, and a food for the sick. The form of the food or drink is not particularly limited. Specific examples thereof include so-called nutraceutical foods or dietary supplements such as a tablet, a granule, a powder, and a health drink. Other examples include drinks such as tea drink, refreshing drink, soda, nutritional drink, fruit juice, and lactic drink; noodles such as buckwheat noodle, wheat noodle, Chinese noodle, and instant noodle; sweets and bakery products such as candy, gum, chocolate, snack, biscuit, jelly, jam, cream, baked goods, and bread; fishery or livestock products such as fish sausage, ham, and sausage; dairy products such as processed milk and fermented milk; fats, oils, and processed foods thereof, such as salad oil, oil for frying, margarine, mayonnaise, shortening, whipped cream, and dressing; seasonings such as sauce and dipping sauce; retort pouch foods such as curry, stew, sauce for rice-bowl cuisine, porridge, and rice soup; and frozen desserts such as ice cream, sherbet, and shaved ice.

The present disclosure provides a heat discoloration suppressant for lecithin, the suppressant containing a fatty acid metal salt. The heat discoloration suppressant for lecithin of the present disclosure may consist of only a fatty acid metal salt or comprise a material other than the fatty acid metal salt. The material other than the fatty acid metal salt is not particularly limited unless it inhibits the heat discoloration suppression effect for lecithin. The heat discoloration suppressant for lecithin of the present disclosure can suppress the heat discoloration of lecithin when added to lecithin, a lecithin preparation, a lecithin-containing oil or fat, a lecithin-containing food additive, a lecithin-containing food, or drink, a lecithin-containing cosmetic, a lecithin-containing medicine, a lecithin-containing feed, a lecithin-containing industrial product or the like.

Moreover, the present invention includes use of a fatty acid metal salt for producing a heat discoloration suppressant for lecithin.

### Examples

The present invention will be described in detail with reference to Examples hereinbelow, but the present invention is not limited to them.

### Example 1: Suppression, by Calcium Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Calcium stearate: (18 carbon atoms, saturated fatty acid metal salt, manufactured by Nitto Chemical Industry Co., Ltd.)

### (2) Experimental method

Rapeseed sirasimeyu (9.76 g) and calcium stearate (0.052 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 22.

### Example 2: Suppression, by Magnesium Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Magnesium stearate: (18 carbon atoms, saturated fatty acid metal salt, manufactured by Nitto Chemical Industry Co., Ltd.)

### (2) Experimental method

Rapeseed sirasimeyu (9.76 g) and Magnesium stearate (0.050 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 25.

### Example 3: Suppression, by Barium Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Barium stearate: (18 carbon atoms, saturated fatty acid metal salt, manufactured by Wako Pure Chemical Industries, Ltd.)

### (2) Experimental method

Rapeseed sirasimeyu (9.76 g) and barium stearate (0.060 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 22.

### Example 4: Suppression, by Sodium Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Sodium stearate: (18 carbon atoms, saturated fatty acid metal salt, manufactured by Wako Pure Chemical Industries, Ltd.)

### (2) Experimental method

Rapeseed sirasimeyu (9.76 g) and sodium stearate (0.026 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 93.

### Example 5: Suppression, by Aluminum Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Aluminum stearate: (18 carbon atoms, saturated fatty acid metal salt, manufactured by NACALAI TESQUE, INC.)

### (2) Experimental method

Rapeseed sirasimeyu (9.76 g) and aluminum stearate (0.075 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 63.

### Example 6: Suppression, by Sodium Caprylate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Sodium caprylate: (8 carbon atoms, saturated fatty acid metal salt, manufactured by Wako Pure Chemical Industries, Ltd.)

### (2) Experimental method

Rapeseed sirasimeyu (9.80 g) and sodium caprylate (0.014 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 22.

### Example 7: Suppression, by Sodium oleate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Sodium oleate: (18 carbon atoms, unsaturated fatty acid metal salt, manufactured by KANTO CHEMICAL CO., INC.)

### (2) Experimental method

Rapeseed sirasimeyu (9.78 g) and sodium oleate (0.026 g) were weighed out and were placed in a 30-ml bottle . The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 22.

### Example 8: Suppression, by Magnesium oleate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Magnesium oleate: (18 carbon atoms, unsaturated fatty acid metal salt, manufactured by KANTO CHEMICAL CO., INC.)

### (2) Experimental method

Rapeseed sirasimeyu (9.76 g) and magnesium oleate (0.050 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 29.

### Example 9: Suppression, by Calcium Oleate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Calcium oleate: (18 carbon atoms, unsaturated fatty acid metal salt, manufactured by KANTO CHEMICAL CO., INC.)

### (2) Experimental method

Rapeseed sirasimeyu (9.76 g) and calcium oleate (0.051 g) were weighed out and were placed in a 30-ml bottle . The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 23.

### Example 10: Suppression, by Calcium Erucate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Erucic acid (22 carbon atoms, unsaturated fatty acid): Palmera A 2290 (trade name, manufactured by KLK OLEO)
Calcium silicate: BRISKOIL CAS-30S (trade name, manufactured by Tomita Pharmaceutical CO., Ltd.)

### (2) Experimental method

Palmera A 2290 (6.55 g) and hexane (23.38 g) were weighed out and were placed in a 70-ml bottle. The Palmera A 2290 was dissolved in the hexane. Moreover, BRISKOIL CAS-30S (7.50 g) was added thereto and the mixture was stirred with heating at a temperature of 60°C for 30 minutes. BRISKOIL CAS-30S was removed by pressure filtration, and the obtained filtrate was concentrated and vacuum-dried (50°C, -0.09 MPa, 5 hours) to obtain a BRISKOIL CAS-30S-treated Palmera A 2290 (1.50 g).

Rapeseed sirasimeyu (9.75 g) and the BRISKOIL CAS-30S-treated Palmera A 2290 (0.061 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 31.

### Comparative Example 1: Without Addition of Fatty Acid Metal Salt (Soybean Lecithin Paste)

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)

### (2) Experimental method

Rapeseed sirasimeyu (9.81 g) and SLP-PASTE (0.19 g) were weighed out and were placed in a 30-ml bottle. The mixture was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 214.

### Comparative Example 2: Addition of Fatty Acid (Caprylic Acid) (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Caprylic acid (8 carbon atoms, saturated fatty acid, manufactured by Wako Pure Chemical Industries, Ltd.)

### (2) Experimental method

Rapeseed sirasimeyu (9.78 g) and caprylic acid (0.026 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 179.

### Comparative Example 3: Addition of Fatty Acid (Stearic Acid)

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Stearic acid (18 carbon atoms, saturated fatty acid, manufactured by Wako Pure Chemical Industries, Ltd.)

### (2) Experimental method

Rapeseed sirasimeyu (9.76 g) and stearic acid (0.052 g) were weighed out and were placed in a 30-ml bottle . The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 162.

### Comparative Example 4: Addition of Fatty Acid (Oleic Acid)

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Oleic acid (18 carbon atoms, unsaturated fatty acid, manufactured by Wako Pure Chemical Industries, Ltd.)

### (2) Experimental method

Rapeseed sirasimeyu (9.76 g) and oleic acid (0.052 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 197.

### Comparative Example 5: Addition of Fatty Acid (Erucic Acid)

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Erucic acid (22 carbon atoms, unsaturated fatty acid): Palmera A 2290 (trade name, manufactured by KLK OLEO)

### (2) Experimental method

Rapeseed sirasimeyu (9.75 g) and erucic acid (0.061 g) were weighed out and were placed in a 30-ml bottle. The mixture was heated at a temperature of 130°C for 10 minutes for dissolution, and was cooled down to a temperature of 60°C or less. After the cooling, SLP-PASTE (0.19 g) was added thereto and was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 206.

### Results of Examples 1 to 10 and Comparative Examples 1 to 5

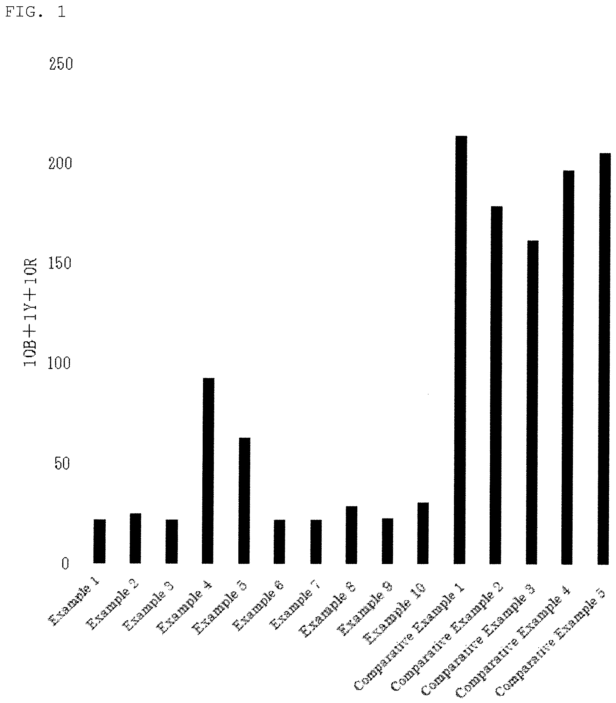

The results of Examples 1 to 10 and Comparative Examples 1 to 5 are shown in FIG. 1. In the case where nothing was added (Comparative Example 1) and the cases where a fatty acid not in the form of a metal salt was added (Comparative Examples 2 to 5), the lecithin turned brownish yellow when heated. In contrast, the results of Examples 1 to 10 show that addition of a fatty acid metal salt suppresses heat discoloration of lecithin. In particular, the values of hues obtained in Examples 1 to 3 and 6 to 10 were one-fifth or less of those obtained in Comparative Examples, showing a significant suppression effect on heat discoloration.

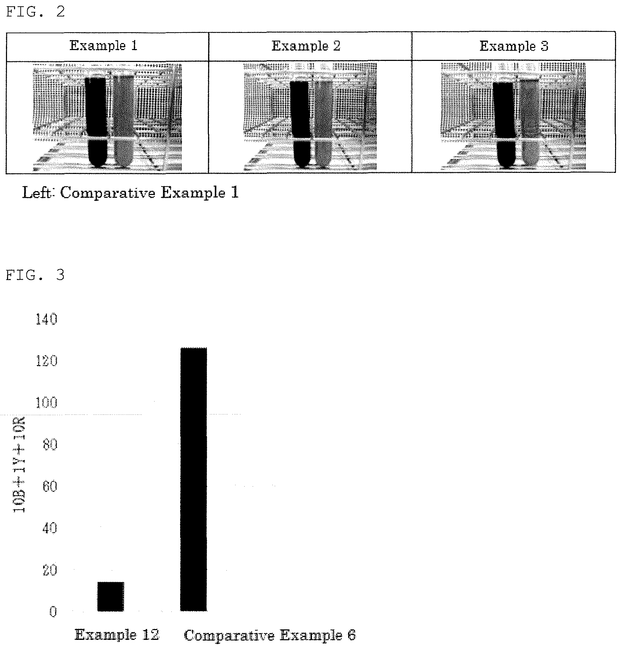

FIG. 2 represents pictures showing the appearances of the samples after the heat discoloration tests in Examples 1 to 3 compared with that in Comparative Example 1. As seen in FIG. 2, discoloration was significantly suppressed in Examples 1 to 3.

### Example 11: Suppression, by Calcium Stearate, of Heat Discoloration of Soybean Lecithin Paste

### (1) Experimental material

Soybean lecithin paste: SLP-PASTE (trade name, manufactured by Tsuji Oil Mills Co., Ltd.)
Rapeseed sirasimeyu (Rapeseed refined oil) (manufactured by Tsuji Oil Mills Co., Ltd.)
Calcium stearate: (18 carbon atoms, saturated fatty acid metal salt, manufactured by Nitto Chemical Industry Co., Ltd.)

### (2) Experimental method

SLP-PASTE (38.00 g) and calcium stearate (10.40 g) were weighed out and were placed in a 140-ml bottle. The mixture was heated at a temperature of 60°C for 10 minutes for dissolution. Then, rapeseed sirasimeyu (9.76 g) and the mixture (0.242 g) of SLP-PASTE and calcium stearate were weighed out and were placed in a 30-ml bottle. The mixture was heated at a temperature of 60°C for 10 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 25.

In Example 1, calcium stearate was dissolved in rapeseed sirasimeyu with heating. After the solution was cooled, SLP-PASTE was added thereto. In contrast, in Example 11, SLP-PASTE and calcium stearate were mixed and then rapeseed sirasimeyu was added to the mixture. The heat discoloration of the sample was suppressed, as was the case in Example 1. That means the timing of adding a fatty acid metal salt does not affect the suppression effect.

### Example 12: Suppression, by Calcium Stearate, of Heat Discoloration of High-Purity Soybean Lecithin Powder

### (1) Experimental material

High-purity soybean lecithin powder: SLP-WHITE (trade name, manufactured by Tsuji Oil Mills Co., Ltd.)
Rapeseed sirasimeyu (Rapeseed refined oil) (manufactured by Tsuji Oil Mills Co., Ltd.)
Calcium stearate: (18 carbon atoms, saturated fatty acid metal salt, manufactured by Nitto Chemical Industry Co., Ltd.)

### (2) Experimental method

Rapeseed sirasimeyu (9.85 g) and calcium stearate (0.052 g) were weighed out and were placed in a 30-ml bottle. The mixture was heated at a temperature of 130°C for 10 minutes for dissolution, and was cooled down to a temperature of 60°C or less. After the cooling, SLP-WHITE (0.10 g) was added thereto and was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 14.

### Comparative Example 6: Without Addition of Fatty Acid Metal Salt (High-Purity Soybean Lecithin Powder)

### (1) Experimental material

High-purity soybean lecithin powder: SLP-WHITE (trade name, manufactured by Tsuji Oil Mills Co., Ltd.)
Rapeseed sirasimeyu (Rapeseed refined oil) (manufactured by Tsuji Oil Mills Co., Ltd.)

### (2) Experimental method

Rapeseed sirasimeyu (9.90 g) and SLP-WHITE (0.10 g) were weighed out and were placed in a 30-ml bottle. The mixture was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 126.

### Comparison of Example 12 and Comparative Example 6

The results of Example 12 and Comparative Example 6 are shown in Fig. 3. As seen in Fig. 3, it was shown that addition of a fatty acid metal salt suppresses heat discoloration of lecithin also in the case where a high-purity soybean lecithin powder is used, as is the case with a soybean lecithin paste.

### Example 13: Suppression, by Calcium Stearate, of Heat Discoloration of PC-Concentrated Soybean Lecithin Paste

### (1) Experimental material

PC-concentrated soybean lecithin paste: SLP-PC35 (trade name, manufactured by Tsuji Oil Mills Co., Ltd.)
Rapeseed sirasimeyu (Rapeseed refined oil) (manufactured by Tsuji Oil Mills Co., Ltd.)
Calcium stearate: (18 carbon atoms, saturated fatty acid metal salt, manufactured by Nitto Chemical Industry Co., Ltd.)

### (2) Experimental method

Rapeseed sirasimeyu (9.76 g) and calcium stearate (0.052 g) were weighed out and were placed in a 30-ml bottle. The mixture was heated at a temperature of 130°C for 10 minutes for dissolution, and was cooled down to a temperature of 60°C or less. After the cooling, SLP-PC35 (0.19 g) was added thereto and was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 16.

### Comparative Example 7: Without Addition of Fatty Acid Metal Salt (PC-Concentrated Soybean Lecithin Paste)

### (1) Experimental material

PC-concentrated soybean lecithin paste: SLP-PC35 (trade name, manufactured by Tsuji Oil Mills Co., Ltd.)
Rapeseed sirasimeyu (Rapeseed refined oil) (manufactured by Tsuji Oil Mills Co., Ltd.)

### (2) Experimental method

Rapeseed sirasimeyu (9.81 g) and SLP-PC35 (0.19 g) were weighed out and were placed in a 30-ml bottle. The mixture was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 138.

### Comparison of Example 13 and Comparative Example 7

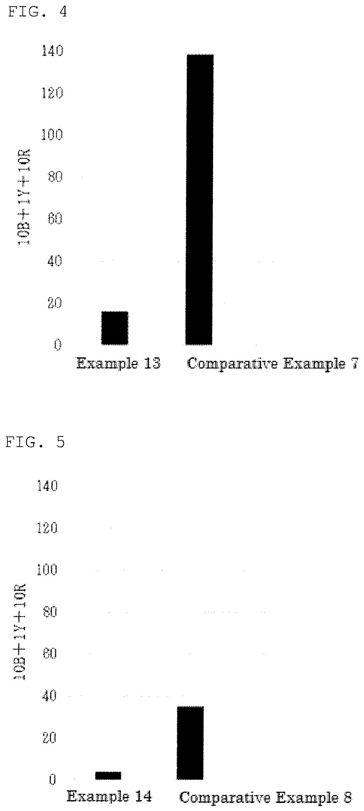

The results of Example 13 and Comparative Example 7 are shown in Fig. 4. As seen in Fig. 4, it was shown that addition of a fatty acid metal salt suppresses heat discoloration of lecithin also in the case where a PC-concentrated soybean lecithin paste is used, as is the case with a soybean lecithin paste.

### Example 14: Suppression, by Calcium Stearate, of Heat Discoloration of PC-Concentrated Soybean Lecithin Lump

### (1) Experimental material

PC-concentrated soybean lecithin lump: SLP-PC70 (trade name, manufactured by Tsuji Oil Mills Co., Ltd.)
Rapeseed sirasimeyu (Rapeseed refined oil) (manufactured by Tsuji Oil Mills Co., Ltd.)
Calcium stearate: (18 carbon atoms, saturated fatty acid metal salt, manufactured by Nitto Chemical Industry Co., Ltd.)

### (2) Experimental method

Rapeseed sirasimeyu (9.85 g) and calcium stearate (0.052 g) were weighed out and were placed in a 30-ml bottle. The mixture was heated at a temperature of 130°C for 10 minutes for dissolution, and was cooled down to a temperature of 60°C or less. After the cooling, SLP-PC70 (0.10 g) was added thereto and was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 4.

### Comparative Example 8: Without Addition of Fatty Acid Metal Salt (PC-Concentrated Soybean Lecithin Lump)

### (1) Experimental material

PC-concentrated soybean lecithin lump: SLP-PC70 (trade name, manufactured by Tsuji Oil Mills Co., Ltd.)
Rapeseed sirasimeyu (Rapeseed refined oil) (manufactured by Tsuji Oil Mills Co., Ltd.)

### (2) Experimental method

Rapeseed sirasimeyu (9.90 g) and SLP-PC70 (0.10 g) were weighed out and were placed in a 30-ml bottle. The mixture was heated at a temperature of 60°C for 30 minutes for dissolution. The prepared sample (6 g) was placed in a test tube and was heated at a temperature of 200°C for 15 minutes. The sample after the heating was measured for hues in accordance with "Standard Methods for the Analysis of Fats, Oils and Related Materials, 2.2.1.1-1996, Color (Lovibond Method)".

### (3) Experimental results

The obtained values of hues were assigned to the formula: "10 × B + 1 × Y + 10 × R"
to give a numerical value. The numerical value was 35.

### Comparison of Example 14 and Comparative Example 8

The results of Example 14 and Comparative Example 8 are shown in Fig. 5. As seen in Fig. 5, it was shown that addition of a fatty acid metal salt suppresses heat discoloration of lecithin also in the case where a PC-concentrated soybean lecithin lump is used, as is the case with a soybean lecithin paste.

The present invention is not limited to each of the embodiments and examples described above and can be variously modified within the scope of claims. The technical scope of the present invention encompasses embodiments obtained by appropriately combining different technical means disclosed in respective embodiments.

## Claims

1. A method for suppressing heat discoloration of lecithin, comprising adding a fatty acid metal salt to lecithin.

2. The method for suppressing heat discoloration according to claim 1, wherein the metal constituting the fatty acid metal salt is at least one selected from the group consisting of lithium, beryllium, sodium, magnesium, aluminum, potassium, calcium, iron, cobalt, nickel, copper, zinc, silver, barium, thallium, and lead.

3. The method for suppressing heat discoloration according to claim 1 or 2, wherein the fatty acid of the fatty acid metal salt is at least one selected from saturated fatty acids and unsaturated fatty acids having 3 to 36 carbon atoms.

4. Use of a fatty acid metal salt for producing a heat discoloration suppressant for lecithin.

## Patentansprüche

1. Verfahren zum Unterdrücken einer Wärmeentfärbung von Lecithin, aufweisend Zugeben eines Fettsäuremetallsalzes zu Lecithin.

2. Verfahren zum Unterdrücken einer Wärmeentfärbung gemäß Anspruch 1, wobei das Metall, das das Fettsäuremetallsalz beinhaltet, mindestens eines ausgewählt unter der Gruppe bestehend aus Lithium, Beryllium, Natrium, Magnesium, Aluminium, Kalium, Calcium, Eisen, Cobalt, Nickel, Kupfer, Zink, Silber, Barium, Thallium und Blei ist.

3. Verfahren zum Unterdrücken einer Wärmeentfärbung gemäß Anspruch 1 oder 2, wobei die Fettsäure des Fettsäuremetallsalzes mindestens eine ausgewählt unter gesättigten Fettsäuren und ungesättigten Fettsäuren mit 3 bis 36 Kohlenstoffatomen ist.

4. Verwendung eines Fettsäuremetallsalzes zum Herstellen eines die Wärmeentfärbung von Lecithin unterdrückenden Mittels.

## Revendications

1. Procédé de suppression de la décoloration à la chaleur de la lécithine, comprenant l'ajout d'un sel métallique d'acide gras à la lécithine.

2. Procédé de suppression de la décoloration à la chaleur selon la revendication 1, dans lequel le métal constituant le sel métallique d'acide gras est au moins l'un choisi dans le groupe constitué des lithium, béryllium, sodium, magnésium, aluminium, potassium, calcium, fer, cobalt, nickel, cuivre, zinc, argent, baryum, thallium et plomb.

3. Procédé de suppression de la décoloration à la chaleur selon la revendication 1 ou 2, dans lequel l'acide gras du sel métallique d'acide gras est au moins l'un choisi parmi des acides gras saturés et des acides gras insaturés ayant 3 à 36 atomes de carbone.

4. Utilisation d'un sel métallique d'acide gras pour produire un suppresseur de décoloration à la chaleur pour la lécithine.
